# EUROPEAN PATENT APPLICATION

(11) **EP 4 609 788 A1**
(43) Date of publication of application: **03.09.2025**
(21) Application number: 23882555.8
(22) Date of filing: 20.10.2023
(51) Int. Cl.: A61B 5/16, A61B 5/02, A61B 5/024, A61B 5/352

(54) **INFORMATION PROCESSING DEVICE, INFORMATION PROCESSING METHOD, AND PROGRAM**

(30) Priority: 24.10.2022 JP 2022169964
(71) Applicant: Suntory Holdings Limited, Osaka 530-8203 (JP)
(72) Inventor: MIZUTANI, Haruo, Kyoto 6190284 (JP)
(74) Representative: Vossius & Partner Patentanwälte Rechtsanwälte mbB
(86) International application number: PCT/JP2023/038000
(87) International publication number: WO 2024/090350

(57) **Abstract**

In order to solve the problem that it is difficult to acquire information on a future status of a user, an information processing apparatus 100 includes: a biological information acquiring unit 143 that acquires time-series information on a status of a living body based on measurement results of a biological signal of a user; a prediction information acquiring unit 145 that acquires prediction information on future time-series information on the user, using learning information configured using time-series information acquired from measurement results of a biological signal in the past, and the time-series information; and a future information output unit 147 that outputs future information on a future status of the user based on the prediction information. Accordingly, it is possible to acquire information on a future status of a user.

## Description

### Technical Field

The present invention relates to an information processing apparatus, an information processing method, and a program that are capable of acquiring information on a future status of a user.

### Background Art

In recent years, users have become increasingly aware of their own health, and various devices and services have been provided to assist users in understanding their health status. For example, Patent Document 1 below discloses a system configured to determine a health risk signal of a user based on an aggregate heartbeat value determined according to biological measurement data obtained from the user, and to provide the signal to the user.

Furthermore, Patent Document 2 discloses a relaxation level determination apparatus that can easily and accurately determine the relaxation level by using the heartbeat (pulse) signal of an examinee who is a user.

It is known that various rhythms affect human sleeping and waking. Such rhythms include, for example, a rhythm called circadian rhythm (circadian cycle). The so-called two-process rhythm is also widely known as the human sleep-wake rhythm (see Non-Patent Document 1 below, for example).

Furthermore, in addition to the conventionally known Transformer, a method called Informer has been proposed as a machine learning method that can be used for prediction of future time-series data, as described in Non-Patent Document 2 below.

### Citation List

### Patent Documents

Patent Document 1: Japanese Patent No. 6531161
Patent Document 2: JP H9-70399A

### Non-Patent Documents

Non-Patent Document 1: Daan S, Beersma DG, Borbely AA. Timing of human sleep: recovery process gated by a circadian pacemaker. Am J Physiol. 1984; 246 (2 Pt 2): R161-83.
Non-Patent Document 2: Zhou, Haoyi, et al. "Informer: Beyond efficient transformer for long sequence time series forecasting." Proceedings of the AAAI Conference on Artificial Intelligence. Vol. 35. No. 12.

### Summary of Invention

### Technical Problem

In the case of evaluating the health status of a user or considering measures to improve the health status, it is considered useful to acquire information on a future status of the user. However, it has been conventionally difficult to acquire information on a future status of a user.

It is an object of the present invention to provide an information processing apparatus, an information processing method, and a program that are capable of acquiring information on a future status of a user.

### Solution to Problem

A first aspect of the present invention is directed to an information processing apparatus including: a biological information acquiring unit that acquires time-series information on a status of a living body based on measurement results of a biological signal of a user; a prediction information acquiring unit that acquires prediction information on future time-series information on the user, using learning information configured using time-series information acquired from measurement results of a biological signal in the past, and the time-series information; and a future information output unit that outputs future information on a future status of the user based on the prediction information.

With this configuration, it is possible to acquire information on a future status of a user.

Furthermore, a second aspect of the present invention is directed to the information processing apparatus according to the first aspect, wherein the prediction information acquiring unit includes a pre-processing unit that performs predetermined filter processing on the time-series information, and the prediction information acquiring unit acquires the prediction information, using the time-series information after the processing by the pre-processing unit.

With this configuration, it is possible to acquire accurate information on a future status of a user.

Furthermore, a third aspect of the present invention is directed to the information processing apparatus according to the second aspect, wherein the filter processing is singular spectrum analysis.

With this configuration, it is possible to acquire accurate information on a future status of a user.

Furthermore, a fourth aspect of the present invention is directed to the information processing apparatus according to the third aspect, wherein the pre-processing unit is configured to acquire the time-series information after the processing, using results having degrees up to a predetermined highest degree obtained through the singular spectrum analysis, and the predetermined degree is 1 or higher and 10 or lower.

With this configuration, it is possible to acquire accurate information on a future status of a user.

Furthermore, a fifth aspect of the present invention is directed to the information processing apparatus according to any one of the first to fourth aspects, wherein the prediction information acquiring unit acquires the prediction information using a machine learning method, using the learning information and the time-series information.

With this configuration, it is possible to acquire accurate information on a future status of a user.

Furthermore, a sixth aspect of the present invention is directed to the information processing apparatus according to any one of the first to fifth aspects, wherein the learning information constitutes a neural network model with an encoder and a decoder using Transformer.

With this configuration, it is possible to acquire accurate information on a future status of a user.

Furthermore, a seventh aspect of the present invention is directed to the information processing apparatus according to the sixth aspect, wherein the decoder of the neural network model is configured not to perform autoregression.

With this configuration, it is possible to more easily acquire accurate information on a future status of a user.

Furthermore, an eighth aspect of the present invention is directed to the information processing apparatus according to any one of the first to fifth aspects, wherein the learning information constitutes a neural network model using an Informer method, the neural network model being a model with an encoder and a decoder using Transformer without using either a ProbSparse Self Attention method or an Encoder-distilling method.

With this configuration, it is possible to acquire more accurate information on a future status of a user.

Furthermore, a ninth aspect of the present invention is directed to the information processing apparatus according to any one of the first to eighth aspects, wherein the time-series information is information on transition of LF or HF related to heartbeat variability of the user, based on information showing an electrocardiogram of the user.

With this configuration, it is possible to acquire information on a future status of a user from measurement results of information showing an electrocardiogram.

Furthermore, a tenth aspect of the present invention is directed to the information processing apparatus according to any one of the first to ninth aspects, wherein the time-series information is information on transition of LF or HF related to heartbeat variability, and the future information output unit acquires, as the future information, information on a time period during which the user is likely to feel stressed or a time period during which the user is likely to be relaxed in a predetermined period of time, based on the prediction information, and outputs the acquired future information.

With this configuration, it is possible to acquire information on a time period during which the user is likely to feel stressed or a time period during which the user is likely to be relaxed in a predetermined period of time.

Furthermore, an eleventh aspect of the present invention is directed to the information processing apparatus according to any one of the first to eighth aspects, wherein the time-series information is information showing a PPG waveform of the user based on pulse waves of the user.

With this configuration, it is possible to acquire information on a future status of a user from pulse waves.

### Advantageous Effects of Invention

With the information processing apparatus and the like according to the present invention, it is possible to acquire information on a future status of a user.

### Brief Description of Drawings

FIG. 1 is a diagram showing the outline of an information processing system according to an embodiment of the present invention.
FIG. 2 is a block diagram of an information processing apparatus in the embodiment.
FIG. 3 is a block diagram of a terminal device and a measuring device in the embodiment.
FIG. 4 is a diagram illustrating the structure of learning information that is used in the information processing apparatus in the embodiment.
FIG. 5 is a flowchart showing an example of an operation of the information processing apparatus in the embodiment.
FIG. 6 is a flowchart showing an example of prediction information acquiring processing of the information processing apparatus in the embodiment.
FIG. 7 is a diagram illustrating time-series information in a specific example of this embodiment.
FIG. 8 is a diagram showing an example of time-series information before filter processing in a specific example of this embodiment.
FIG. 9 is a diagram showing an example of time-series information after filter processing in a specific example of this embodiment.
FIG. 10 is a diagram showing an example of prediction information acquired in a specific example of this embodiment.
FIG. 11 is a schematic view of a computer system in the foregoing embodiment.
FIG. 12 is a block diagram of the computer system in the embodiment.

### Description of Embodiment

Hereinafter, an embodiment of an information processing apparatus and the like will be described with reference to the drawings. It should be noted that constituent elements denoted by the same reference numerals in the embodiments perform similar operations, and thus a description thereof may not be repeated.

The terms used below are generally defined as follows. The meanings of these terms do not always have to be interpreted as indicated herein, and have to be interpreted in light of individual explanations below, if any, for example.

"Identifier for a matter" is a letter, a symbol, or the like for uniquely identifying the matter. The identifier is an ID, for example, but may be any type of information with which the corresponding matter can be identified. That is to say, the identifier may be the name of the matter itself that it indicates, or symbols that are combined so as to uniquely correspond to the matter.

"Acquiring" may include acquiring a matter that is input by a user or the like, or acquiring information stored in the apparatus or another apparatus (the information may be information stored in advance or information generated through information processing in the apparatus). "Acquiring information stored in another apparatus" may include acquiring information stored in the other apparatus via an API or the like, or acquiring the content of a document file (including the webpage content, etc.) provided by the other apparatus.

Furthermore, a so-called machine learning method may be used to acquire information. A machine learning method may be used as follows, for example. That is to say, a learning model (learning information) in which a particular type of input information is taken as input and a type of output information that is to be acquired is taken as output is configured using a machine learning method. For example, two or more pairs of input information and output information are prepared in advance, the two or more pairs of information are given to a module for configuring a learning model of machine learning to configure a learning model, and the configured learning model is accumulated in a storage unit. The learning model may also be said to be a classifier. There is no limitation on the machine learning method, and examples thereof include deep learning, random forests, and SVM. For example, functions in various machine learning frameworks and various existing libraries, such as fastText, tinySVM, random forest, and TensorFlow, can be used for the machine learning. The acquiring information using such a learning model may also be said to be acquiring information through machine learning.

"Learning model" is not limited to those obtained through machine learning. The learning model may be a table indicating a correspondence relationship between an input vector based on input information or the like and output information, for example. In this case, output information corresponding to a feature vector based on input information may be acquired from the table, or a vector that is close to a feature vector based on the input information may be generated using two or more input vectors in the table and parameters for weighting the input vectors, and output information corresponding to the input vectors and parameters used for the generation may be used to acquire final output information. The acquiring information using such a learning model may also be said to be acquiring information using a correspondence relationship. The learning model may be a function or the like that represents a relationship between an input vector based on the input information or the like and information for generating output information, for example. In this case, for example, information corresponding to a feature vector based on input information may be obtained using a function, and the obtained information may be used to acquire output information. The acquiring information using such a learning model may also be said to be acquiring information using a function.

"Outputting information" is a concept that encompasses display on a display screen, projection using a projector, printing by a printer, output of a sound, transmission to an external apparatus, accumulation in a recording medium, and delivery of a processing result to another processing apparatus or another program. Specifically, for example, this concept encompasses enabling information to be displayed on a webpage, transmission as an email or the like, and outputting information for printing.

"Accepting information" is a concept that encompasses accepting information input from an input device such as a keyboard, a mouse, or a touch panel, receiving information transmitted from another apparatus or the like via a wired or wireless communication line, and accepting information read from a recording medium such as an optical disk, a magnetic disk, or a semiconductor memory.

### Embodiment

In this embodiment, an information processing system is configured using an information processing apparatus. The information processing apparatus is configured to acquire prediction information on a future biological signal of a user who is a provider, from measurement results of a biological signal of the user, and to output future information on a future status of the user based on the prediction information. The information processing apparatus may be called a prediction information output apparatus, and the information processing system may be called a prediction information output system.

Preferably, the information processing apparatus may be configured as follows. For example, the measurement results may be time-series information, and prediction information may be acquired from time-series information after predetermined pre-processing. For example, the pre-processing may be singular spectrum analysis, and time-series information after the processing may be configured based on the results having degrees up to a predetermined highest degree obtained through singular spectrum analysis. For example, the prediction information may be acquired using a neural network model, and in particular, a Transformer model may be used. In this case, the decoders of the Transformer model may be configured not to perform autoregression.

As a specific example, the information processing apparatus may be configured, for example, to use information indicating the transition of LF, HF, or LF/HF based on an electrocardiogram (ECG), as the time-series information. For example, a time period during which the user is likely to feel stressed or a time period during which the user is likely to be relaxed in a predetermined period of time may be specified from the prediction information, and information indicating that time period may be output.

Hereinafter, an example of the information processing system using the thus configured information processing apparatus will be described.

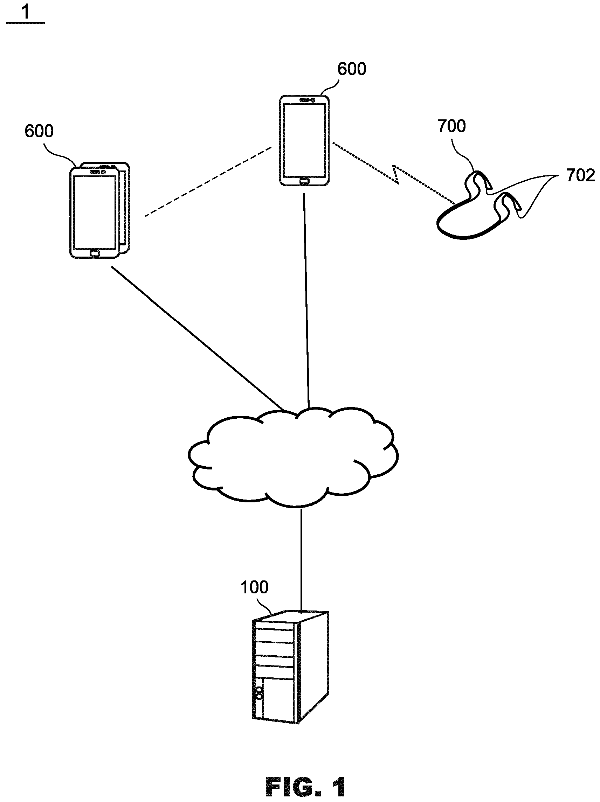

FIG. 1 is a diagram showing the outline of an information processing system 1 according to an embodiment of the present invention.

In this embodiment, the information processing system 1 includes an information processing apparatus 100, terminal devices 600, and measuring devices 700. The information processing apparatus 100 and the terminal devices 600 can communicate with each other, for example, via a network such as a local area network or the Internet. A measuring device 700 and a terminal device 600 can communicate with each other. The configuration of the information processing system 1 is not limited to this. There is no limitation on the number of apparatuses of each type included in the information processing system 1, and other types of apparatuses may also be included in the information processing system 1.

Each measuring device 700 is, for example, a so-called wearable device. The measuring device 700 has electrode units 702 that are in contact with a body surface of the user. The measuring device 700 is configured to be able to measure biological signals related to bioelectricity such as action potentials and resting potentials of the body of the user via the electrode units 702. In this embodiment, the measuring device 700 is configured to be able to measure time-series biological signals such as brain waves and pulse waves.

The measuring device 700 may be configured to be able to measure the body temperature or the like of the user by means of the electrode units 702 or other unshown units. The measuring device 700 may include a measuring unit for measuring biological signals of the user instead of the electrode units 702 or in addition to the electrode units 702. The measuring unit may be configured to be able to detect a target different from the bioelectricity. For example, the measuring unit may be able to receive light emitted or reflected from the body of the user to obtain time-series measurement results. That is to say, the measuring device 700 may be configured, for example, to be able to receive light emitted or reflected from the body of the user and to measure information on the body of the user based on the results thereof. The measuring device 700 includes, for example, a storage unit (not shown) in which measurement results, control programs for the measuring device 700, and the like are stored, a processing unit (not shown) that performs various processing operations using the information stored in the storage unit, a transmitting and receiving unit (not shown) for transmitting and receiving information, and the like.

The measuring device 700 is, for example, an ear-mounted headset that can be worn by the user at all times. The measuring device 700 is configured such that, when worn, two electrode units 702 are in contact with the vicinity of the left and right mastoid processes of the user, thereby enabling measurement of the brain waves and the pulse waves of the user. Such a configuration of the measuring device 700 is known, and thus a detailed description thereof has been omitted. The measuring device 700 is not limited to a headset type, and may be in various forms that allow the user to continue wearing it, such as eyeglasses, wristwatch, ring, neck-hanging, belt, and clothing types. The measuring device 700 may include a sound output unit (not shown) that outputs a user-hearable sound by vibrating air or an object, a display screen (not shown) that can be viewed by the user, and the like. The measuring device 700 may be configured to be able to output information to the user by means of the sound output unit, the display screen, and the like. The measuring device 700 may be two or more physically separated apparatuses that can cooperate to measure one or more biological signals. The measuring device 700 may include electrode units 702 that are used in contact with the body surface of the user only during the measurement. The measuring device 700 is not limited to such wearable devices, but may also be those that are used by the user to measure biological signals under certain circumstances, such as, for example, a blood pressure monitor, a thermometer, a scale, an electroencephalograph, or an electrocardiograph.

In this embodiment, the measurement results of time-series biological signals and information based thereon are referred to as measurement information on a status of a living body. The measurement information may be, for example, raw data of measurement results acquired by measuring biological signals, or information obtained by converting or processing the raw data. The measurement information may also be said to be time-series information that is time-series information on a status of a living body based on measurement results of a biological signal of a user. That is to say, time-series information is measurement results of time-series biological signals, or time-series information acquired based thereon.

In this embodiment, examples of the measurement results of a biological signal include, but are not limited to, ECG (electrocardiogram), pulse waves such as PPG (photoplethysmogram), brain waves, and the like.

The measuring device 700 in this embodiment is connected wirelessly or via a wired connection to the terminal device 600, and is configured to be able to transmit and receive information to and from the terminal device 600. For example, the measuring device 700 is configured to be able to perform short-range wireless communication with the terminal device 600. The measuring device 700 is configured, for example, to be able to transmit the measurement results to the terminal device 600. The measurement results can be transmitted to the terminal device 600 as time-series information accumulated by the measuring device 700, or can be transmitted sequentially to the terminal device 600. The terminal device 600 is configured to be able to perform various types of processing using information received from the measuring device 700, and to transmit the received information or information obtained by processing the received information to the information processing apparatus 100. The measuring device 700 itself may be able to be connected to a network such as a local area network or the Internet, and to communicate with the information processing apparatus 100 and the terminal device 600 connected to the network. The measurement results may be transmitted to the information processing apparatus 100. The measuring device 700 may be configured to be able to perform operations such as measuring biological signals or performing predetermined processing on measurement results by itself, or may be configured to be able to perform these operations in conjunction with the terminal device 600 by transmitting and receiving signals.

The users of the information processing system 1 can use the information processing system 1 by using the terminal devices 600 and the measuring devices 700. In FIG. 1, for example, portable information terminal devices such as so-called smartphones are shown as the terminal devices 600, but those that are used as the terminal devices 600 are not limited to such portable information terminal devices. For example, personal computers (PC) such as laptop computers may be used as the terminal devices 600, or other devices such as tablet-type information terminal devices may be used. In the examples below, a description will be given assuming that portable information terminal devices such as smartphones are used as the terminal devices 600, but there is no limitation to this.

The terminal devices 600 may include built-in measuring devices 700.

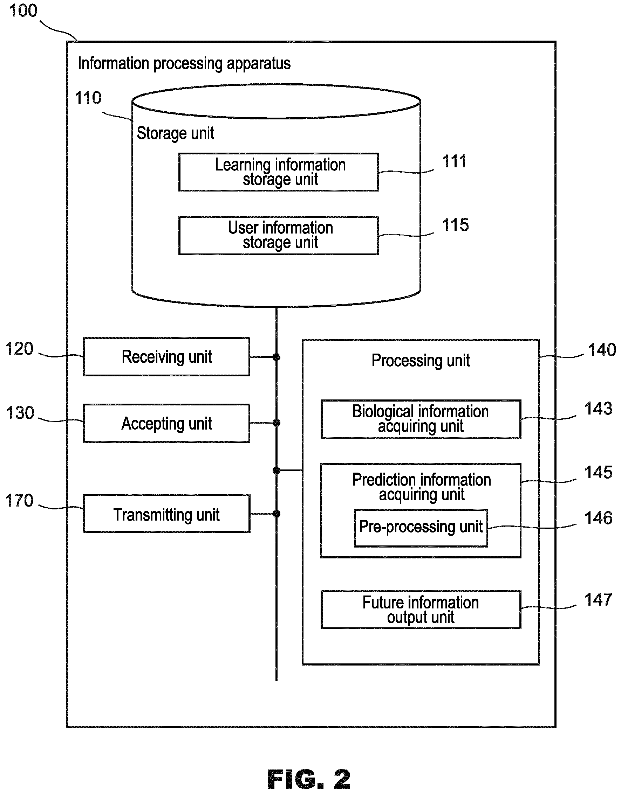

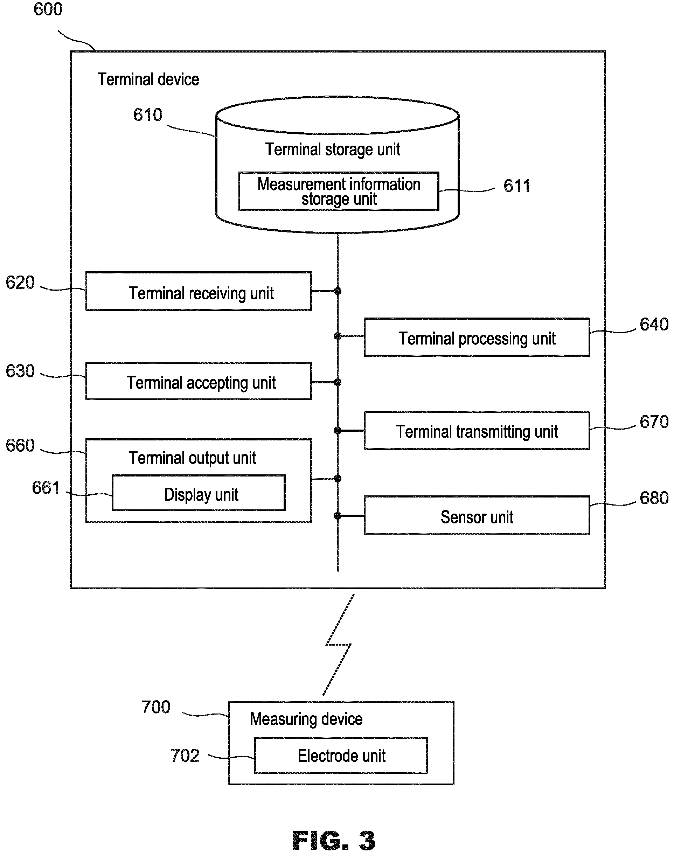

FIG. 2 is a block diagram of the information processing apparatus 100. FIG. 3 is a block diagram of a terminal device 600 and a measuring device 700.

As shown in FIG. 2, the information processing apparatus 100 includes a storage unit 110, a receiving unit 120, an accepting unit 130, a processing unit 140, and a transmitting unit 170. The information processing apparatus 100 is, for example, a server apparatus.

The storage unit 110 includes a learning information storage unit 111 and a user information storage unit 115.

Learning information acquired in advance is stored in the learning information storage unit 111. In this embodiment, the learning information is generated using a so-called machine learning method. The learning information is, for example, generated and stored in the learning information storage unit 111 by the processing unit 140 as described later, but there is no limitation to this. That is to say, learning information generated in an apparatus different from the information processing apparatus 100 may be stored in the learning information storage unit 111. Details of the learning information will be described later.

In this embodiment, the learning information is configured for each user targeted for biological signal measurement. Learning information corresponding to each user is generated and stored in the learning information storage unit 111. Each piece of learning information is stored in association with a user identifier for identifying a user. However, there is no limitation to this, and learning information that can be commonly used for two or more users may be prepared. Learning information may be prepared for each group of users who share common attributes. In this case, for example, learning information may be stored in association with an identifier for identifying a group.

User information is stored in the user information storage unit 115. In this embodiment, the user information is information in which a user identifier, which is an identifier for identifying a user who uses the information processing system 1, is associated with information on the user. The user information may contain various types of information. For example, the user information may contain information transmitted from the terminal device 600 that is used by the user, information on the user acquired by the information processing apparatus 100 as described later, and the like. The information transmitted from the terminal device 600 that is used by the user corresponds to, for example, time-series information, information input by the user to the terminal device 600, and the like. The information on the user acquired by the information processing apparatus 100 corresponds to, for example, information on the sleep-wake rhythm of the user and prediction information based thereon, as will be described later, and the like. User information transmitted from other external apparatuses or the like may be stored in the user information storage unit 115.

Time-series information transmitted from the terminal devices 600 of the respective users is stored in the user information storage unit 115. The time-series information is, for example, measurement results obtained by the users performing measurement using the measuring devices 700, and is information stored in terminal storage units 610 of the terminal devices 600. The procedure in which the time-series information is stored in the user information storage unit 115 is not limited to this. The configuration is also possible in which time-series information prepared in advance is stored in the user information storage unit 115 and that time-series information is used.

The receiving unit 120 receives information transmitted from other apparatuses. The receiving unit 120 stores the received information, for example, in the storage unit 110. In this embodiment, for example, the users perform input of information and the like and transmit the information to the information processing apparatus 100, using the terminal devices 600. The receiving unit 120 can store each piece of transmitted information in the storage unit 110 in association with the user identifier. In this embodiment, the receiving unit 120 receives the time-series information transmitted from the terminal devices 600, and stores the information in the storage unit 110 in association with the user identifiers. In the case in which the receiving unit 120 receives these pieces of information from the terminal devices 600, the receiving unit 120 can specify user identifiers of the users pertaining to the transmission based on the transmitted information.

The accepting unit 130 accepts information input using an unshown input part connected to the information processing apparatus 100. The accepting unit 130 stores the accepted information, for example, in the storage unit 110. The information may be input by any input part such as a numeric keypad, a keyboard, a mouse, or a menu screen. The accepting unit 130 may accept information input through an input operation performed using a reading device (e.g., code reader, etc.) connected to the information processing apparatus 100 (e.g., including information read by the device).

The accepting unit 130 may be considered to accept information received by the receiving unit 120, as information input to the information processing apparatus 100. That is to say, the input of information to the information processing apparatus 100 may be construed to mean that the information is indirectly input to the information processing apparatus 100 by the user via the terminal device 600 or the like or that the information is directly input to the information processing apparatus 100 by the user using an input part. The processing in which the user causes the information processing apparatus 100 to execute a program for automatically generating information or to function by giving various types of information to a program, thereby causing information to be given to the information processing apparatus 100 may be considered as the input of information to the information processing apparatus 100.

The processing unit 140 includes a biological information acquiring unit 143, a prediction information acquiring unit 145, and a future information output unit 147. In this embodiment, the prediction information acquiring unit 145 includes a pre-processing unit 146. The processing unit 140 performs various types of processing. The various types of processing are, for example, processing that is performed by the units of the processing unit 140 as follows.

The biological information acquiring unit 143 acquires time-series information for a user targeted for prediction. In this embodiment, the biological information acquiring unit 143 acquires the time-series information transmitted from the terminal device 600 of the user and received by the receiving unit 120, from the user information storage unit 115. The biological information acquiring unit 143 acquires time-series information on a user targeted for processing, from the user information storage unit 115, based on the user identifier of the user.

The prediction information acquiring unit 145 acquires prediction information on future time-series information on a user, using time-series information on the user and learning information corresponding to the user. The acquired prediction information is, for example, stored in the storage unit 110. The term "future" refers to, for example, a period or point in time later than the end of the period of time-series information acquired for a user targeted for prediction, and in particular refers to a period or point in time that includes a point in time later than the point in time when the prediction information is acquired.

The prediction information is acquired using a machine learning method. That is to say, in this embodiment, the learning information is configured using a machine learning method using time-series information acquired from measurement results of a biological signal in the past (which may also be said to be past time-series information). The past time-series information may be information on time-series measurement results themselves, or information obtained using the time-series measurement results. The prediction information is information obtained by predicting information that temporally follows the past time-series information. The prediction information may also be said to be prediction results of time-series information in a predetermined period of time later than the end of the period of past time-series information.

The learning information is information that is used to configure a learning model in which past time-series information is taken as input information and prediction information on future time-series information is taken as output information. For example, the processing unit 140 can generate the learning information as follows, for example. That is to say, the processing unit 140 configures, using a machine learning method, learning information in which past time-series information is taken as input and prediction information on future time-series information is taken as output. For example, two or more pairs of past time-series information and output values, for learning, are prepared in advance, learning information is configured by giving the two or more pairs of information to a module for configuring learning information of machine learning, and the configured learning information is accumulated in the learning information storage unit 111. Examples of the machine learning method include, but are not limited to, those using recursive neural networks (RNN) such as long- and short-term memory. For example, functions in various machine learning frameworks and various existing libraries, such as TensorFlow and PyTorch, can be used for the machine learning. The pairs of information for learning may be prepared using results obtained by measuring each user in advance. Such learning information may be generated by an apparatus different from the information processing apparatus 100.

In this embodiment, the prediction information acquiring unit 145 is configured to acquire prediction information using time-series information after the processing by the pre-processing unit 146. That is to say, the learning information as described above is generated using, as input, past time-series information after the processing. Examples of the processing by the pre-processing unit 146 include, but are not limited to, those described later.

The pre-processing unit 146 performs predetermined filter processing on time-series information acquired by the biological information acquiring unit 143 (referred to as pre-processing information), thereby acquiring time-series information after the processing. In other words, the pre-processing unit 146 performs dimensionality reduction (dimensionality compression) on the pre-processing information. The pre-processing unit 146 is configured, for example, to perform known singular spectrum analysis, as the predetermined filter processing. Accordingly, prediction information can be acquired using time-series information after the processing in which oscillating and trend components are extracted. Such filter processing can be performed, for example, using a numerical calculation library such as NumPy, for example.

The pre-processing unit 146 is configured to acquire time-series information after the processing, using the results having degrees up to a predetermined highest degree obtained through singular spectrum analysis. The predetermined degree is, for example, 1 or higher and 10 or lower, and preferably 5 or higher and 10 or lower. In this embodiment, the pre-processing unit 146 is configured to acquire time-series information after the processing, using the results having degrees up to a highest degree of 10 obtained through singular spectrum analysis.

The method by which the pre-processing unit 146 performs dimensionality reduction is not limited to this. For example, the pre-processing unit 146 may be configured to perform dimensionality reduction of pre-processing information using an autoencoder using a convolutional neural network. The dimensionality reduction using an autoencoder can be configured using known methods. For example, the pre-processing information may be input to a probabilistic encoder to extract vectors indicating latent oscillating components and vectors indicating latent trend components, respectively.

The future information output unit 147 outputs future information on a future status of a user targeted for prediction, based on the prediction information. The future information may be, for example, prediction information itself, or information acquired based on the prediction information. Examples of the information acquired based on the prediction information may include, but are not limited to, a condition or status of the user at a future point in time or during a future period of time, expressed in a predetermined classification, and a score. For example, the information may be information on comparison results obtained through comparison between a reference value of each user obtained from past measurement information and prediction information, or information on comparison results obtained through comparison between a predetermined reference value commonly used for multiple users and prediction information.

In this embodiment, the future information output unit 147 may be configured to acquire, as future information, information on a time period during which a user is likely to feel stressed or a time period during which the user is likely to be relaxed in a predetermined period of time, based on the prediction information, and to output the acquired future information. Examples of the predetermined period of time include, but are not limited to, the period of the prediction information. For example, the future information output unit 147 specifies a time period that satisfies a predetermined determination condition based on the prediction information, and acquires information on the specified time period, as future information. For example, a case will be assumed in which the prediction information relates to time-series information indicating sympathetic and parasympathetic activities, respectively. Examples of such prediction information include, but are not limited to, information on LF and HF related to heartbeat variability and information on brain wave activity. In such cases, for example, the predetermined determination condition can be set to be a state in which either sympathetic or parasympathetic activity is dominant over the other. Also, for example, the predetermined determination condition can be set to be a state in which the degree of dominance of either sympathetic or parasympathetic activity over the other is greater than or equal to a predetermined degree.

The output of future information is performed, for example, by transmitting information from the transmitting unit 170 to the terminal device 600 of the user, but there is no limitation to this. For example, the output of future information may be a hand-off to processing that is performed by the information processing apparatus 100. For example, the output may be performed by displaying the prediction information in the form of text or images on a display screen provided by the information processing apparatus 100. The output of future information may also mean accumulating the future information in the storage unit 110 to perform these processes.

The transmitting unit 170 transmits information to other devices constituting the information processing system 1 via a network. The transmitting unit 170 transmits information, for example, to the terminal device 600. In other words, the transmitting unit 170 outputs information, for example, to the terminal device 600.

Next, the configuration of a terminal device 600 will be described.

As shown in FIG. 3, the terminal device 600 includes a terminal storage unit 610, a terminal receiving unit 620, a terminal accepting unit 630, a terminal processing unit 640, a terminal output unit 660, a terminal transmitting unit 670, and a sensor unit 680. The terminal output unit 660 includes a display unit 661.

Various types of information are accumulated in the terminal storage unit 610. The terminal storage unit 610 includes a measurement information storage unit 611. For example, measurement information measured by the measuring device 700 is accumulated in the measurement information storage unit 611. Also, future information transmitted from the information processing apparatus 100 is accumulated in the terminal storage unit 610.

The terminal receiving unit 620 receives information transmitted from the information processing apparatus 100, the measuring device 700, and the like, via a network. The terminal receiving unit 620 accumulates the received information, for example, in terminal storage unit 610 such that the information can be acquired by the terminal processing unit 640 and the like.

The terminal accepting unit 630 accepts various input operations performed on the terminal device 600 by a user who uses the terminal device 600. The operations are performed using, for example, an unshown input device, but there is no limitation to this.

The terminal processing unit 640 performs various information processing operations using the units of the terminal device 600. For example, future information transmitted from the information processing apparatus 100 is output by the terminal output unit 660 to the user. This allows the user to learn about the future information.

The terminal output unit 660 outputs information by, for example, displaying it on the display unit 661 that is a display device. The method for outputting information is not limited to this, and may also be performed by outputting a sound or the like from a speaker or the like.

The terminal transmitting unit 670 transmits information acquired by the terminal processing unit 640 or the like, for example, via a network.

Examples of the sensor unit 680 include, but are not limited to, a microphone, an acceleration sensor, a barometric pressure sensor, and the like. The sensor unit 680 may be used to measure a biological signal of the user. The sensor unit 680 may include electrodes that can measure a biological signal of the user.

In this case, machine learning methods that may be used in this embodiment will be described.

As learning information, information is used that can constitute neural network models with encoders and decoders using so-called Transformer. Such neural network models called Transformer are known models used to predict time-series information, but may be partially modified. Transformer-based neural network models may be said to have the encoder-decoder structure with a self-attention mechanism. Transformer-based neural network models may be said to have the encoder-decoder structure using a multi-head attention mechanism. In this embodiment, the learning information is a neural network model that uses, in part, the so-called Informer method as described in Non-Patent Document 2 above. Informer may be said to be a derivative neural network model of Transformer. The decoders of the neural network model that is constituted by the learning information are configured not to perform autoregression. That is to say, the learning information is a neural network model that uses the so-called Informer method in part with a generative-style decoder structure that does not involve autoregression, and that constitutes a model with encoders and decoders using standard Transformer. The learning information can constitute a neural network model with encoders and decoders in which ProbSparse Self Attention and Encoder-distilling methods are not used among the methods called Informer.

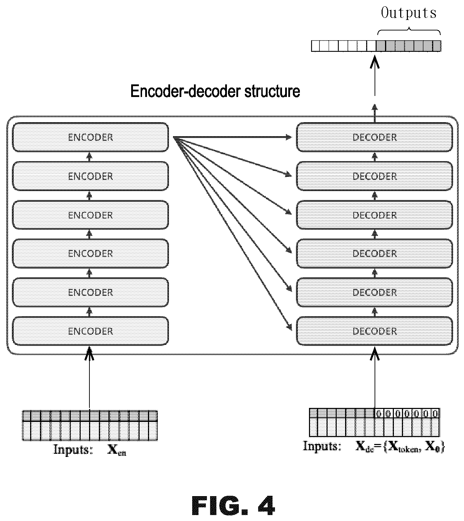

FIG. 4 is a diagram illustrating the structure of learning information that is used in the information processing apparatus 100.

The drawing schematically shows the encoder-decoder structure of the learning information. Generative-style decoders can be described as follows. That is to say, in standard Transformer, the input data corresponding to the sequence of data output by the decoders is missing in the last part and is shifted backward as a whole, and only one first piece of data is padded. In contrast, in generative-style decoders, as shown in the drawing, all the portions of the input data corresponding to the sequence of data to be output are padded in the inference unit, so that the output data portion is generated at once during inference.

With this configuration using generative-style decoders of Informer, it is possible to acquire prediction information at higher speed.

Next, an example of the operation of the information processing apparatus 100 performed when a user uses the information processing system 1 according to this embodiment will be described. In this embodiment, the user can use the information processing system 1, for example, by operating a predetermined application on the terminal device 600 while accessing the information processing apparatus 100 via the terminal device 600 or receiving information transmitted from the information processing apparatus 100. The predetermined application may be, for example, a dedicated application that operates using information transmitted from the information processing apparatus 100, a web browser that displays a web application provided by the information processing apparatus 100 such that the application can be used, or the like.

In this embodiment, the information processing system 1 is typically used as follows. That is to say, the user measures his or her own biological signals while wearing the measuring device 700 over a predetermined period of time. The time-series information obtained from the measurement is made to be transferred to the terminal device 600. The user then transmits the time-series information stored in the terminal storage unit 610 from the terminal device 600 to the information processing apparatus 100. Accordingly, the information processing apparatus 100 acquires the prediction information and outputs the future information. The terminal device 600 receives the output future information and causes the terminal output unit 660 to display the information on the display device based on the future information. This allows the user to check his or her own future information and information based thereon.

In the case in which such operations of the information processing system 1 are performed, the information processing apparatus 100 performs various operations as follows, for example. These operations are performed by the processing unit 140 executing control operations and the like while using the units.

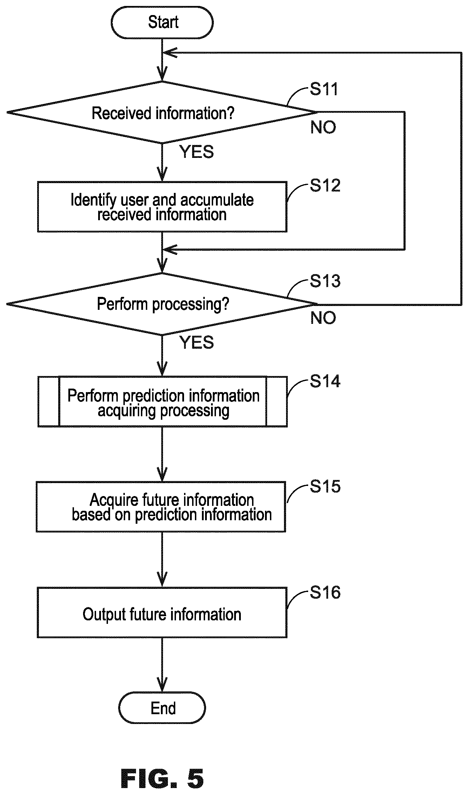

FIG. 5 is a flowchart showing an example of an operation of the information processing apparatus 100.

(Step S11) The processing unit 140 determines whether or not the receiving unit 120 has received information transmitted from the terminal device 600 or the like. If it is determined that it has received the information, the procedure advances to step S12, or otherwise the procedure advances to step S13.

(Step S12) The processing unit 140 identifies the user based on the received information, and accumulates the received information in the user information storage unit 115 in association with the user identifier. For example, if time-series information is transmitted from the terminal device 600 in association with a user identifier, the processing unit 140 accumulates the received time-series information in the user information storage unit 115 in association with that user identifier.

(Step S13) The processing unit 140 determines whether or not a trigger to perform processing such as acquiring prediction information or outputting future information has occurred. In other words, the processing unit 140 determines whether or not a condition for starting processing such as acquiring prediction information or outputting future information has been satisfied. If it is determined that such a trigger has occurred, the procedure advances to step S14. Otherwise, the procedure returns to step S11.

In this case, for example, the above-mentioned trigger can be the provision of an instruction from the user through the terminal device 600 to perform processing such as acquiring prediction information or outputting future information. For example, the above-mentioned trigger can be the transmission of predetermined information corresponding to such an instruction through the terminal device 600, or the like. The trigger is not limited to these. For example, the above-mentioned trigger can be the arrival of a predetermined time, and in this case, the future information can be output periodically. For example, the trigger can be the fulfillment of various conditions such as the new receipt of time-series information or other information.

(Step S14) The processing unit 140 causes the prediction information acquiring unit 145 to perform prediction information acquiring processing. The prediction information acquiring processing will be described later. The prediction information on the user is acquired and accumulated in the user information storage unit 115 through the prediction information acquiring processing.

(Step S15) The processing unit 140 causes the future information output unit 147 to acquire future information based on the prediction information.

(Step S16) The processing unit 140 causes the future information output unit 147 to output the future information. In this embodiment, the processing unit 140 outputs the acquired future information to the target user. That is to say, the processing unit 140 causes the transmitting unit 170 to output the future information to the terminal device 600 that is used by the target user. This allows the terminal device 600 that has received the future information to display the future status of the user.

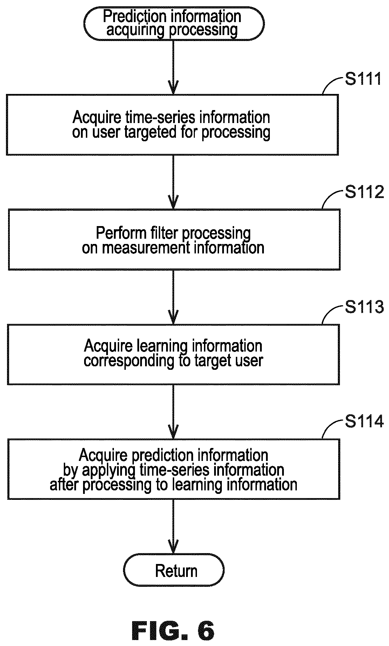

FIG. 6 is a flowchart showing an example of the prediction information acquiring processing of the information processing apparatus 100.

(Step S111) The prediction information acquiring unit 145 acquires time-series information on the target user, from the user information storage unit 115, based on the user identifier of the user.

(Step S112) The prediction information acquiring unit 145 causes the pre-processing unit 146 to perform filter processing on the measurement information.

(Step S113) The prediction information acquiring unit 145 acquires learning information corresponding to the user identifier of the target user, from the learning information storage unit 111.

(Step S114) The prediction information acquiring unit 145 applies the time-series information after the processing by the pre-processing unit 146 to the learning information, thereby acquiring prediction information. The acquired prediction information is accumulated in the storage unit 110 in association with the user identifier of the target user. Thereafter, the procedure returns to the processing in FIG. 4.

Next, specific examples of the acquisition of prediction information by the information processing apparatus 100 according to this embodiment will be described.

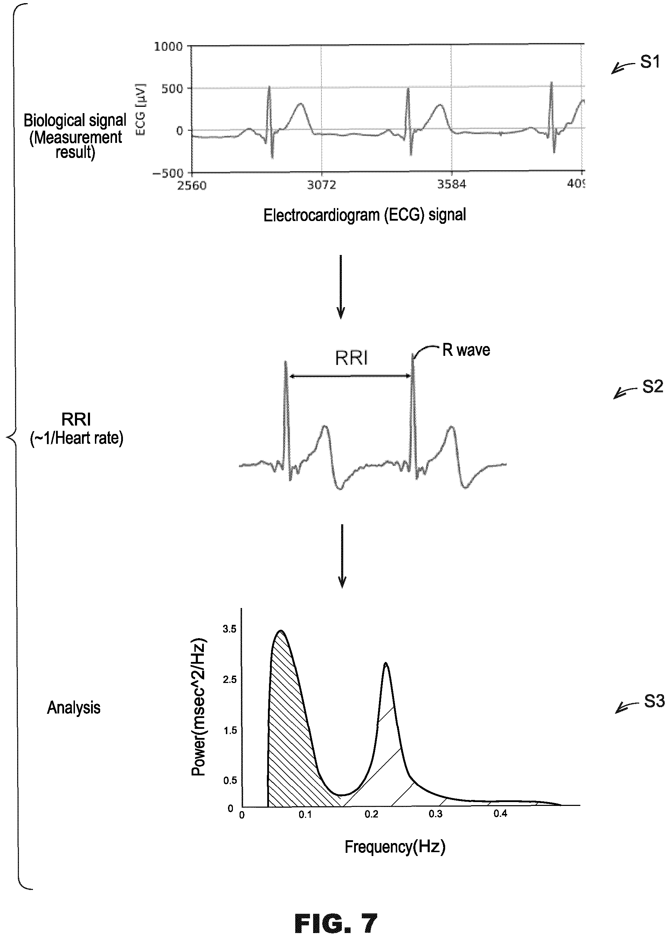

FIG. 7 is a diagram illustrating time-series information in a specific example of this embodiment.

In this specific example, the time-series information is information acquired based on the information showing the ECG of a user as measurement results. The time-series information is information indicating the transition of LF and HF related to heartbeat variability of a user. The time-series information may be information on the transition of LF/HF (ratio of LF to HF). As the prediction information, for example, prediction of the transition of LF or HF over a predetermined period of time in the future may be obtained, or prediction of the transition of LF/HF may be obtained.

LF and HF are values obtained by integrating the power spectrum obtained by frequency analysis of the transition of heartbeat intervals in the respective frequency domain bands (low-frequency band and high-frequency band). LF can be used as an indicator of the degree of activation of the user's sympathetic nervous system, and HF can be used as an indicator of the degree of activation of the user's parasympathetic nervous system. That is to say, LF, HF, and LF/HF can be used as information on the degree of activation of the user's autonomic nervous system.

As shown in the drawing, if the electrocardiogram signal for a predetermined measurement period (e.g., 5 minutes) is obtained as a measurement result (S1), the RRIs (heartbeat intervals: intervals between R waves) is obtained therefrom (S2). LF and HF can be obtained based on the power spectrum obtained by frequency analysis of the transition of RRIs over the predetermined measurement period (S3). LF and HF obtained for each predetermined measurement period can be summarized as time-series information.

Such acquisition of time-series information from the ECG may be performed, for example, by the terminal device 600, but it may also be performed by the measuring device 700 or by the information processing apparatus 100 that acquired the measurement results.

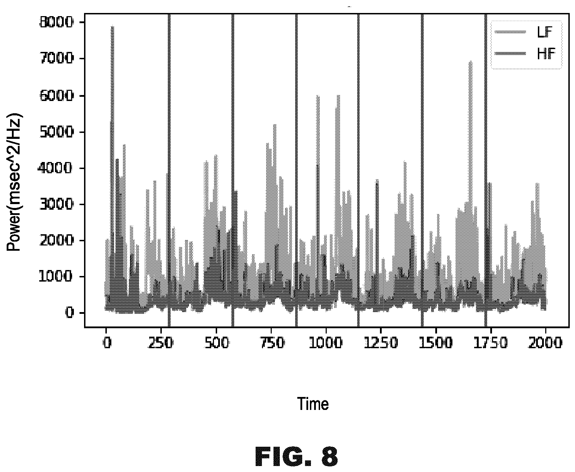

FIG. 8 is a diagram showing an example of time-series information before filter processing in a specific example of this embodiment.

The drawing shows time-series information indicating the transition of LF and HF over time, which is obtained as described above. In this drawing and the next drawing, for example, time-series information for approximately one week is shown. If such time-series information is filtered by the pre-processing unit 146, the result is as follows.

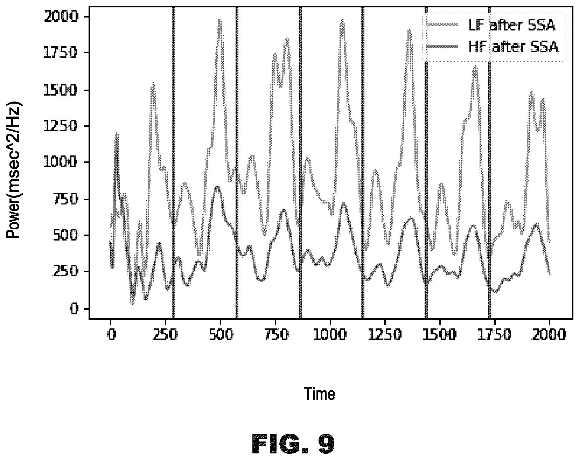

FIG. 9 is a diagram showing an example of time-series information after filter processing in a specific example of this embodiment.

The drawing shows time-series information after the processing obtained by restoring the time-series information, using the results having degrees up to a highest degree of 10 obtained through singular spectrum analysis, in contrast to the previous drawing. Such time-series information after the processing is information that well represents the transition of LF and HF of the user, with the principal components of the time-series information well extracted. By using such time-series information after the processing, it is possible to acquire accurate prediction information.

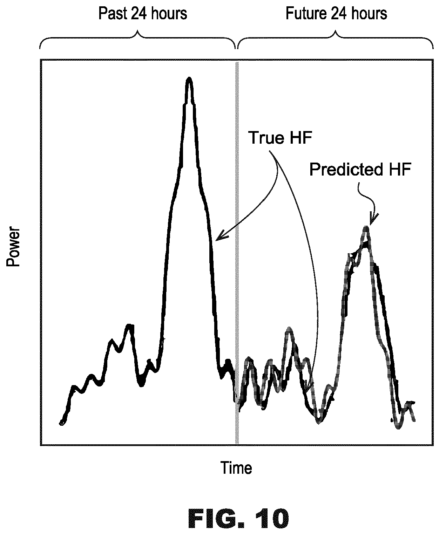

FIG. 10 is a diagram showing an example of prediction information acquired in a specific example of this embodiment.

The drawing shows prediction information on HF (predicted HF) for a predetermined period of time (e.g., 24 hours) in the future obtained from the time-series information on HF (true HF) for a predetermined period of time (e.g., 24 hours) in the past. The time-series information based on the actual subsequent measurement results is also shown for the predetermined time in the future. In this manner, it is possible to acquire prediction information, which is future time-series information, from past time-series information. The prediction information is an accurate prediction of the transition of actual time-series information.

In the thus obtained prediction information, the time period during which HF is dominant over LF by a predetermined degree in a predetermined period of time in the future may be output as future information indicating a time period during which the target user is likely to feel stressed (a time period during which the user is unlikely to be relaxed). The time period during which HF is dominant over LF by a predetermined degree in a predetermined period of time in the future may be output as future information indicating a time period during which the target user is likely to be relaxed (a time period during which the user is unlikely to feel stressed).

As another specific example, instead of using the information constituting the above-described electrocardiogram as measurement results, the time-series information obtained from the pulse waves as the measurement results may be used to obtain prediction information on the user. For example, it is also possible to acquire time-series information indicating the transition of LF and HF and the like from the transition of PPIs (pulse-to-pulse intervals), and acquire prediction information on the transition of LF and HF and the like in the same manner as described above.

Furthermore, it is also possible to acquire information showing the PPG waveform of the user based on the pulse waves of the user as the time-series information, and obtain the future transition of heart rate (HR) and the like of the user based on the time-series information. For example, in the case of obtaining the transition of heart rate, for example, data for learning containing a PPG waveform and an ECG waveform that is paired therewith is prepared, and information indicating the transition of heart rate obtained from the ECG waveform is used so as to be paired with the corresponding PPG waveform, to configure learning information of machine learning. The transition of heart rate can be obtained by applying the time-series information based on measurement results to such learning information. As such learning information, information is used that can constitute a neural network model in which the structure employed in Transformer is used as the encoder structure. This allows the PPG waveform, which can be obtained relatively easily, to be used to acquire prediction information on heart rate with accuracy similar to that in the case in which the information is obtained from ECG. The learning information may be configured such that the instantaneous value of the heart rate can be obtained. The prediction information may be acquired by obtaining the transition of values of indicators other than the heart rate. It is possible to output, for example, future information on the user's peripheral nerve activity, from such prediction information.

Furthermore, it is also possible to obtain prediction information on future brain waves of the user, by acquiring information showing brain waves as time-series information. The thus obtained prediction information may be used to output future information on the activation of the user's central nervous system. For example, information useful for the user can be obtained by appropriately predicting the degree of brain arousal during sleep and the degree of brain arousal during waking.

As explained above, the information processing apparatus 100 can acquire prediction information on future time-series information for an individual user, from time-series information on a status of a living body. Accordingly, it is possible to acquire information on the future status of the user. The user can provide the time-series information to the information processing apparatus 100, for example, by measuring biological signals in a simple method using the measuring device 700 that is a wearable device. Accordingly, the user can easily obtain information on his or her future state. In this embodiment, prediction information is acquired using learning information that constitutes a neural network model using Transformer. Accordingly, it is easy to acquire accurate prediction information.

The storage unit 110 and the terminal storage unit 610 described above are preferably non-volatile recording media, but they may alternately be realized by volatile recording media. Information and the like acquired by each apparatus are stored in the corresponding units, but the procedure in which information and the like are stored is not limited to this. For example, information and the like may be stored via a recording medium, information and the like transmitted via a communication line or the like may be stored, or information and the like input via an input device may be stored.

Furthermore, the processing unit 140 and the terminal processing unit 640 described above may be typically realized by MPUs, memories, or the like. Typically, the processing procedure of the processing unit 140 and the terminal processing unit 640 is realized by software, and the software is stored in a recording medium such as a ROM. Note that the processing procedure may be realized by hardware (dedicated circuits).

Furthermore, the information that can be accepted by the accepting unit 130 or the terminal accepting unit 630 may be input by any input part such as a numeric keypad, a keyboard, a mouse, or a menu screen. The accepting unit 130 and the terminal accepting unit 630 may be realized by device drivers for an input part such as a numeric keypad or a keyboard, control software for a menu screen, or the like.

Furthermore, the receiving unit 120 and the terminal receiving unit 620 are typically realized by wireless or wired communication parts, but they may also be realized by broadcast receiving parts.

Furthermore, the transmitting unit 170 and the terminal transmitting unit 670 are typically realized by wireless or wired communication parts, but they may also be realized by broadcasting parts.

The information processing apparatus 100 may be constituted by a single server, multiple servers that operate in cooperation with each other, or a computer or other device built into other equipment. It will be appreciated that the servers may be so-called cloud servers, ASP servers, or the like, and there is no limitation on the type thereof.

The processing in this embodiment may be realized by software. The software may be distributed by software downloads or the like. Furthermore, the software may be distributed in a form where the software is stored in a recording medium such as an optical disk. The software that realizes the information processing apparatus 100 in this embodiment is the following sort of program. Specifically, this program is a program that is executed on a computer of the information processing apparatus 100, the program causing the computer of the information processing apparatus 100 to function as: a biological information acquiring unit that acquires time-series information on a status of a living body based on measurement results of a biological signal of a user; a prediction information acquiring unit that acquires prediction information on future time-series information on the user using a machine learning method, using learning information configured using time-series information acquired from measurement results of a biological signal in the past, and the time-series information; and a future information output unit that outputs future information on a future status of the user based on the prediction information.

### Others

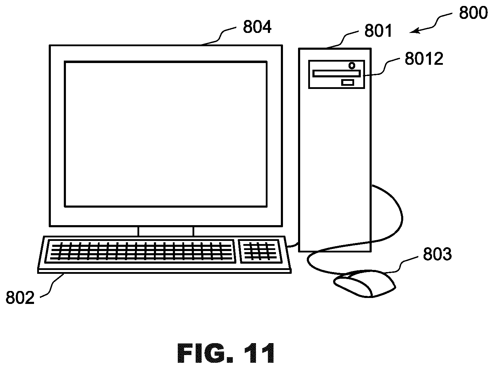

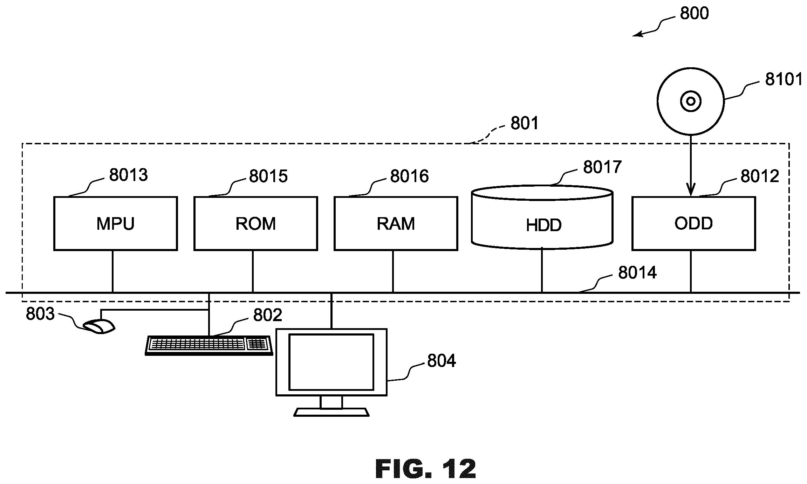

FIG. 11 is a schematic view of a computer system 800 in the foregoing embodiment. FIG. 12 is a block diagram of the computer system 800 in the embodiment.

These drawings show a configuration example of a computer that executes the programs described in this specification to realize the information processing apparatus and the like in the foregoing embodiment. The foregoing embodiment may be realized using computer hardware and a computer program executed thereon.

The computer system 800 includes a computer 801 including an optical disk drive, a keyboard 802, a mouse 803, and a monitor 804.

The computer 801 includes, in addition to the optical disk drive (ODD) 8012, an MPU 8013, a bus 8014 connected to the optical disk drive 8012 and the like, a ROM 8015 in which a program such as a boot up program is stored, a RAM 8016 that is connected to the MPU 8013 and is a memory in which a command of an application program is temporarily stored and a temporary storage area is provided, and a hard disk (HDD) 8017 in which programs such as an application program and a system program and data are stored. Although not shown, the computer 801 may further include a network card that provides connection to a LAN.

Each program for causing the computer system 800 to execute the functions of the information processing apparatus and the like in the foregoing embodiment may be stored in a optical disk 8101 that is inserted into the optical disk drive 8012, and be transmitted to the hard disk 8017. Alternatively, the program may be transmitted via a network (not shown) to the computer 801 and stored in the hard disk 8017. At the time of execution, the program is loaded into the RAM 8016. The program may be loaded from the optical disk 8101, or may be loaded directly from a network.

The program does not necessarily have to include, for example, an operating system (OS) or a third party program to cause the computer 801 to execute the functions of the information processing apparatus and the like in the foregoing embodiment. The program may only include a command portion to call an appropriate function (module) in a controlled mode and obtain desired results. The manner in which the computer system 800 operates is well known, and thus a detailed description thereof has been omitted.

It should be noted that, in the program, in a transmitting step of transmitting information, a receiving step of receiving information, or the like, processing that is performed only hardware such as processing that is performed by a modem or an interface card in the transmitting step (processing that can be performed only by hardware) is not included.

Furthermore, the computer that executes the program may be constituted by a single computer, or constituted by multiple computers. That is to say, centralized processing may be performed, or distributed processing may be performed.

Furthermore, in the foregoing embodiment, two or more constituent elements in one apparatus may be physically realized by one medium.

Furthermore, in the foregoing embodiment, each constituent element may be configured by dedicated hardware, or alternatively, constituent elements that can be realized by software may be realized by executing a program. For example, each constituent element may be realized by a program execution unit such as a CPU reading and executing a software program stored in a recording medium such as a hard disk or a semiconductor memory. At the time of executing the program, the program execution unit may execute the program while accessing the storage unit or the recording medium. Furthermore, this program may be executed by downloading from a server or the like, or may be executed by reading a program stored in a predetermined recording medium (e.g., an optical disk, a magnetic disk, a semiconductor memory, etc.). Furthermore, the program may be used as a program for constituting a program product. Furthermore, the computer that executes the program may be constituted by a single computer, or constituted by multiple computers. That is to say, centralized processing may be performed, or distributed processing may be performed, respectively.

In the foregoing embodiment, each process (function) may be realized as centralized processing using a single apparatus (system), or may be realized as distributed processing using multiple apparatuses (in this case, the entire system constituted by multiple apparatuses that perform distributed processing may be regarded as one "apparatus").

Furthermore, in the foregoing embodiment, information transmission performed between constituent elements may be such that, for example, if two constituent elements for transmitting information are physically different from each other, the transmission is performed by one of the constituent elements outputting the information and the other constituent element accepting the information, or alternatively, if two constituent elements for transmitting information are physically the same, the transmission is performed by shifting from a processing phase corresponding to one of the constituent elements to a processing phase corresponding to the other constituent element.

Furthermore, in the foregoing embodiment, information related to the processing that is performed by each constituent element, for example, information that is to be accepted, acquired, selected, generated, transmitted, or received by each constituent element, information such as a threshold value, a numerical expression, or an address used by each constituent element in the processing and the like may be retained in an unshown recording medium temporarily or for a long period of time even if not specified in the description above. Furthermore, the information may be accumulated in the unshown recording medium by each constituent element or by an unshown accumulating unit. Furthermore, the information may be read from the unshown recording medium by each constituent element or by an unshown reading unit.

Furthermore, in the foregoing embodiment, if information used by each constituent element or the like, for example, information such as a threshold value, an address, or various setting values used by each constituent element in the processing may be changed by a user, the user may be or may not be allowed to change such information as appropriate even if not specified in the description above. If the user is allowed to change such information, the change may be realized by, for example, an unshown accepting unit that accepts a change instruction from the user and an unshown changing unit that changes information according to the change instruction. The unshown accepting unit may accept the change instruction, for example, by accepting information from an input device, by receiving information transmitted via a communication line, or by accepting information read from a predetermined recording medium.

The present invention is not limited to the embodiment set forth herein. Various modifications are possible within the scope of the invention.

An embodiment may be configured by combining constituent elements of the foregoing embodiment or modified examples as appropriate. For example, the configuration of the foregoing embodiment is not limited to those described above, and constituent elements of the foregoing embodiment or modified examples may be replaced by or combined with constituent elements of other embodiments or the like as appropriate. Some of the constituent elements or the functions may be omitted in the foregoing embodiment or modified examples.

### Industrial Applicability

As described above, the information processing apparatus according to the present invention makes it possible to acquire information on a future status of a user, thus rendering this apparatus useful as an information processing apparatus and the like.

### List of Reference Numerals

- 1: Information processing system
- 100: Information processing apparatus
- 110: Storage unit
- 111: Learning information storage unit
- 115: User information storage unit
- 120: Receiving unit
- 130: Accepting unit
- 140: Processing unit
- 143: Biological information acquiring unit
- 145: Prediction information acquiring unit
- 146: Pre-processing unit
- 147: Future information output unit
- 170: Transmitting unit
- 600: Terminal device
- 610: Terminal storage unit
- 620: Terminal receiving unit
- 630: Terminal accepting unit
- 640: Terminal processing unit
- 660: Terminal output unit
- 661: Display unit
- 670: Terminal transmitting unit
- 680: Sensor unit
- 700: Measuring device
- 702: Electrode unit

## Claims

1. An information processing apparatus comprising:
a biological information acquiring unit that acquires time-series information on a status of a living body based on measurement results of a biological signal of a user;
a prediction information acquiring unit that acquires prediction information on future time-series information on the user, using learning information configured using time-series information acquired from measurement results of a biological signal in the past, and the time-series information; and
a future information output unit that outputs future information on a future status of the user based on the prediction information.

2. The information processing apparatus according to claim 1,
wherein the prediction information acquiring unit includes a pre-processing unit that performs predetermined filter processing on the time-series information, and
the prediction information acquiring unit acquires the prediction information, using the time-series information after the processing by the pre-processing unit.

3. The information processing apparatus according to claim 2, wherein the filter processing is singular spectrum analysis.

4. The information processing apparatus according to claim 3,
wherein the pre-processing unit is configured to acquire the time-series information after the processing, using results having degrees up to a predetermined highest degree obtained through the singular spectrum analysis, and
the predetermined degree is 1 or higher and 10 or lower.

5. The information processing apparatus according to claim 1 or 2, wherein the prediction information acquiring unit acquires the prediction information using a machine learning method, using the learning information and the time-series information.

6. The information processing apparatus according to claim 5, wherein the learning information constitutes a neural network model with an encoder and a decoder using Transformer.

7. The information processing apparatus according to claim 6, wherein the decoder of the neural network model is configured not to perform autoregression.

8. The information processing apparatus according to claim 5, wherein the learning information constitutes a neural network model using an Informer method, the neural network model being a model with an encoder and a decoder using Transformer without using either a ProbSparse Self Attention method or an Encoder-distilling method.

9. The information processing apparatus according to claim 1 or 2, wherein the time-series information is information on transition of LF or HF related to heartbeat variability of the user, based on information showing an electrocardiogram of the user.

10. The information processing apparatus according to claim 1 or 2,
wherein the time-series information is information on transition of LF or HF related to heartbeat variability, and
the future information output unit acquires, as the future information, information on a time period during which the user is likely to feel stressed or a time period during which the user is likely to be relaxed in a predetermined period of time, based on the prediction information, and outputs the acquired future information.

11. The information processing apparatus according to claim 1 or 2, wherein the time-series information is information showing a PPG waveform of the user based on pulse waves of the user.

12. An information processing method comprising:
a biological information acquiring step of acquiring time-series information on a status of a living body based on measurement results of a biological signal of a user;
a prediction information acquiring step of acquiring prediction information on future time-series information on the user, using learning information configured using time-series information acquired from measurement results of a biological signal in the past, and the time-series information; and
a future information output step of outputting future information on a future status of the user based on the prediction information.

13. A program for causing a computer to function as:
a biological information acquiring unit that acquires time-series information on a status of a living body based on measurement results of a biological signal of a user;
a prediction information acquiring unit that acquires prediction information on future time-series information on the user, using learning information configured using time-series information acquired from measurement results of a biological signal in the past, and the time-series information; and
a future information output unit that outputs future information on a future status of the user based on the prediction information.
